# EUROPÄISCHE PATENTSCHRIFT

(11) **EP 1 289 577 B1**
(45) Veröffentlichungstag und Bekanntmachung des Hinweises auf die Patenterteilung: **28.02.2007**
(21) Anmeldenummer: 01945213.5
(22) Anmeldetag: 30.05.2001
(51) Int. Cl.: A61M 1/00, A61B 17/3203, A61M 3/02

(54) **FETTABSAUGVORRICHTUNG**
LIPOSUCTION DEVICE
DISPOSITIF DE LIPOSUCCION

(30) Priorität: 31.05.2000 DE 20009786 U
(43) Veröffentlichungstag der Anmeldung: 12.03.2003
(73) Patentinhaber: Taufig, Ahmmed Ziah, 50668 Köln (DE)
(72) Erfinder: Taufig, Ahmmed Ziah, 50668 Köln (DE)
(74) Vertreter: Selting, Günther
(86) Internationale Anmeldenummer: PCT/EP2001/006142
(87) Internationale Veröffentlichungsnummer: WO 2001/091827

(56) Entgegenhaltungen:
- EP-A- 0 331 313
- EP-A- 0 657 150
- WO-A-98/06446
- WO-A-99/22656
- DE-U- 20 009 786
- US-A- 4 941 872
- US-A- 5 135 482
- US-A- 5 336 170
- US-A- 5 807 313
- US-A- 5 968 008

## Beschreibung

Die Erfindung bezieht sich auf eine Fettabsaugvorrichtung zum Entfernen von Unterhautfett.

Zum operativen Entfernen von Unterhautfett werden Fettabsaugvorrichtungen eingesetzt, die im wesentlichen aus einer Absaugkanüle zum Absaugen des Unterhautfettes bestehen. Die Absaugkanüle wird mit ihrem freien Ende durch die Haut hindurchgestochen, so dass anschließend das Fett unterhalb der Haut durch eine Absaugöffnung am freie Ende der Absaugkanüle abgesaugt werden kann. Um das Fett besser absaugen zu können, wird vor dem Absaugvorgang mit einer Einspritzvorrichtung eine Arbeitsflüssigkeit in das Unterhautfettgewebe eingespritzt, die das Unterhautfett bzw. das Fettgewebe löst. Aus US-A-5 968 008 ist eine Fettabsaugvorrichtung bekannt, bei der die Absaugkanüle und die Einspritzleitung in einem Bauteil zusammenfasst sind. Die Einspritzöffnung zum Einspritzen der Arbeitsflüssigkeit befindet sich am vorderen Ende der Einspritzleitung. Die Einspritzöffnung ist klein und punktartig und liegt genau in der Längsachse der Einspritzleitung. Der aus der Einspritzöffnung austretende Arbeitsflüssigkeitsstrahl besteht entweder aus einem gebündelten strichförmigen Flüssigkeitsstrahl oder aus einem sich großflächig aber drucklos verteilenden Wasserstrahl, der ein großes Raumvolumen ausfüllt. Der Arbeitsflüssigkeitsstrahl bewirkt also entweder einen rein punktartigen Gewebeabtrag oder aber eine großflächige Verteilung eines kraftlosen Arbeitsflüssigkeitstrichters auf das Unterhautfett bzw. Gewebe.

Die Druckschrift DE 200 09 786 U1 ist vorveröffentlicht in Bezug auf den Anmeldetag der vorliegenden europäischen Patentanmeldung, und offenbart eine Fettabsaugvorrichtung mit einer eine Absaugöffnung aufweisenden Absaugkanüle und einer Einspritzleitung mit einer Einspritzöffnung zum Einspritzen einer Arbeitsflüssigkeit, wobei die Einspritzöffnung am vorderen distalen Ende der Einspritzleitung angeordnet und schlitzförmig ausgebildet ist, so dass die Arbeitsflüssigkeit in Form eines Fächers austritt. Der fächerförmige Arbeitsflüssigkeitsstrahl tritt axial aus der Einspritzöffnung aus. Mit einem axial austretenden Flüssigkeitsfächer ist es unter Umständen schwierig, mit der Arbeitsflüssigkeit tief in das zu entfernende Unterhaut-Gewebe einzudringen. Die Fettabsaugvorrichtung hat die Form einer geradlinigen Lanze und wird durch eine relativ kleine Öffnung in der Haut praktisch parallel zu der Haut in das Unterhaut-Gewebe eingeführt. Die Fettabsaug-Lanze kann mehr oder weniger nur in einer Ebene parallel zur Haut bewegt werden. Da der Flüssigkeitsstrahl stets axial nach vorne gerichtet ist, muss die Fettabsauglanze als ganzes so geneigt werden, dass sie wenigstens in einem spitzen Winkel von mindestens 10 - 20 ° zur Hautebene geneigt ist, damit der Flüssigkeitsfächer unter diesem Winkel in das UnterhautFettgewebe eindringen kann. Das Anstellen der Fettabsaug-Lanze in einem Winkel von mindestens 10 - 20° ist umständlich, kraftraubend, jedenfalls an der Hautöffnung traumatisierend und erlaubt kein genaues Arbeiten und Abtragen des Unterhaut-Fettgewebes.

Aufgabe der Erfindung ist es demgegenüber, eine Fettabsaugvorrichtung zu schaffen, die das Entfernen von Unterhautfett erleichtert.

Diese Aufgabe wird erfindungsgemäß mit den Merkmalen des Anspruchs 1 gelöst.

Die Einspritzöffnung ist erfindungsgemäß am vorderen Einspritzleitungsende angeordnet und schlitzförmig ausgebildet, so dass ein kraftvoller Arbeitsflüssigkeits-Fächer austritt. Mit dem fächerförmigen Arbeitsflüssigkeitsaustritt lässt sich ein größeres Volumen von Unterhautfettgewebe gezielt, und dennoch gleichmäßig und zügig von der Haut lockern bzw. abschälen. Dadurch wird ein gleichförmiger Abtrag von Unterhautfett gewährleistet und die Entstehung von Hohlräumen oder Ausbeulungen durch ungleichmäßigen Gewebeabtrag vermieden. Die Breite des Einspritzöffnungs-Schlitzes ist kleiner als 1,0 mm, insbesondere kleiner als 0,1 mm. Die Länge des Schlitzes beträgt, je nach Schlitzbreite, einen bis mehrere Millimeter. Durch die geringe Schlitzbreite wird ein relativ geringer Arbeitsflüssigkeitsverbrauch bei gleichzeitig hoher Abtragleistung sichergestellt. Der geringe Arbeitsflüssigkeitsverbrauch von unter 10l/h hat zum einen den Vorteil, dass die Fettabsaugvorrichtung einen geringeren Arbeitsflüssigkeitsverbrauch hat und damit entsprechend kleiner ausgelegt werden kann. Der geringe Arbeitsflüssigkeitsverbrauch hat fernen den medizinisch wichtigen Vorteil, dass auch nur eine relativ kleiner Menge von Absaugflüssigkeit in den betreffenden Unterhautfett-Bereich eingespritzt wird und durch die Absaugkanüle wieder abgesaugt werden muss. Das Absaugen kleinerer Mengen von Arbeitsflüssigkeits-Fettgemisch ist einfacher zu kontrollieren, so dass unerwünschtes Aufblähen von Unterhautfett-Bereichen durch Arbeitsflüssigkeit vermieden wird.

Die Einspritzöffnung ist in einem Winkel zwischen 3° und 45° geneigt zu der Längsachse der Einspritzleitung angeordnet, so dass der Arbeitsflüssigkeits-Fächer unter diesem Winkel zu der Einspritzleitungs-Längsachse austritt. Der Arbeitsflüssigkeits-Fächer ist also nicht parallel zur Einspritzleitungs-Längsachse, sondern hierzu angewinkelt. Durch das Anwinkeln lässt sich der Arbeitsflüssigkeits-Fächer problemlos auf das unter der Hautoberfläche liegende Unterhautfett richten. Hierdurch wird das Abschälen des Unterhautfettes von der Haut erheblich erleichtert.

Vorzugsweise weist die Einspritzleitung an ihrem einspritzöffnungsseitigem Ende eine trichterförmige Beschleunigungsdüse auf. Bevor die Arbeitsflüssigkeit durch die schlitzförmige Einspritzöffnung austritt, wird sie in der Beschleunigungsdüse auf eine hohe Geschwindigkeit beschleunigt. Auf diese Weise wird die Einspritzflüssigkeit bereits kurz vor Erreichen der Einspritzöffnung auf ihre Austrittsgeschwindigkeit beschleunigt. Hierdurch wird die Ausbildung eines präzisen Arbeitsflüssigkeitsfächers realisiert.

Vorzugsweise ist die schlitzartige Einspritzöffnung in einem Winkel von 10° bis 30°, insbesondere von ungefähr 18° geneigt zu der Längsachse der Einspritzleitung angeordnet. Wie Versuche ergeben haben, ist ein Anwinkeln des Arbeitsflüssigkeits-Fächers in einem Winkel zwischen 10° und 30° besonders vorteilhaft für ein effektives Abschälen und Abtragen von Unterhautfett.

Gemäß einer bevorzugten Ausgestaltung ist eine Arbeitsflüssigkeitspumpe zum Pumpen der Arbeitsflüssigkeit zu der Einspritzöffnung vorgesehen, wobei der Ausgangsdruck der Arbeitsflüssigkeitspumpe durch einen Druckwähler einstellbar ist. Der Druck der Arbeitsflüssigkeit, und damit der Druck des austretenden Arbeitsflüssigkeits-Fächers ist damit an die jeweilige Situation anpassbar. Auf diese Weise kann sowohl ein zügiges Abtragen großer Unterhautfett-Volumina realisiert werden, als auch ein vorsichtiges Abtragen dünner Unterhautfett-Schichten erfolgen. Hierdurch wird insbesondere die Traumatisierung von Nicht-Fettgewebe gering gehalten.

Vorzugsweise ist eine Saugpumpe zum Absaugen des Unterhautfettes und eine Regelungsvorrichtung vorgesehen, die die Saugleistung der Saugpumpe in Abhängigkeit von der Pumpleistung der Arbeitsflüssigkeitspumpe regelt. Bei hoher Pumpleitung, d.h. bei großen Mengen eingespritzter Arbeitsflüssigkeit wird die Saugleistung, d.h. die abgepumpte Menge am Arbeitsflüssigkeits-Unterhautfett-Gemisch entsprechend erhöht. Auf diese Weise wird sichergestellt, dass ein Aufblähen der Unterhaut mit Arbeitsflüssigkeit vermieden wird. Gleichzeitig wird sichergestellt, dass das abgelöste Unterhautfett einschließlich der eingespritzten Arbeitsflüssigkeit annähernd vollständig und unmittelbar abgesaugt wird, so dass das Ergebnis der Unterhautfettabtragung sofort sichtbar ist und beurteilt werden kann. Fehleinschätzungen der Frage, wie viel Unterhautfett noch abgetragen werden muss, werden auf diese Weise vermieden.

Gemäß einer bevorzugten Ausgestaltung ist ein Handgriff zum Halten der Absaugkanüle und der Einspritzleitung vorgesehen, wobei an dem Handgriff ein Pumpenschalter zum Schalten der Arbeitsflüssigkeitspumpe vorgesehen ist. Auf diese Weise lässt sich die Funktion der Arbeitsflüssigkeitspumpe bequem und unmittelbar steuern. Hierdurch wird das gezielte Abtragen bestimmter Unterhautfett-Partien erheblich erleichtert.

Vorzugsweise ist an der Absaugkanüle ein Ultraschallgenerator zur Gewebelockerung angeordnet. Der Ultraschallgenerator ist möglichst in der Nähe der Einspritzöffnung angeordnet, um im Zielbereich der eingespritzten Arbeitsflüssigkeit eine möglichst große Lockerungswirkung durch den generierten Ultraschall zu bewirken.

Alternativ oder ergänzend kann an der Absaugkanüle auch ein Laser zur Gewebeerwärmung und -lösung vorgesehen sein. Durch die laserunterstützte Gewebeerwärmung wird die Unterhautfett-Ablösung und -Abtragung vereinfacht.

Gemäß einer bevorzugten Ausgestaltung ist die Einspritzleitung mit der Einspritzöffnung axial beweglich zu dem Handgriff. Ferner ist ein Axialantrieb vorgesehen, der die Einspritzleitung mit der Einspritzöffnung axial oszillierend antreibt. Auf diese. Weise wird eine axial oszillierende Bewegung des Arbeitsflüssigkeits-Fächers realisiert, wodurch ein Verharren des Arbeitsflüssigkeits-Fächers und damit ein unerwünschtes Eingraben des Arbeitsflüssigkeits-Fächers in das Gewebe vermieden wird. Hierdurch wird die Sicherheit gegen zu tiefes Eindringen des Arbeitsflüssigkeits-Fächers in Gewebe erhöht.

Gemäß einer bevorzugten Ausgestaltung ist die Einspritzleitung zentral innerhalb der Absaugkanüle angeordnet. Auf diese Weise wird eine kompakte Konstruktion der Fettabsaugvorrichtung realisiert. Alternativ oder ergänzend kann die Einspritzleitung auch außen an die Absaugkanüle angrenzend montiert sein. Es können auch mehrere Einspritzleitungen um die Absaugkanüle herum angeordnet sein.

Vorzugsweise weist die Absaugkanüle mehrere über den Umfang verteilte Absaugöffnungen auf. Der Mantel der Absaugkanüle ist also über eine bestimmte Länge perforiert ausgebildet. Dadurch lässt sich das. Fett-Arbeitsflüssigkeits-Gemisch schnell absaugen und ist die Absaugfunktion auch dann noch gewährleistet, wenn eine Absaugöffnung verstopft sein sollte.

Vorzugsweise ist die Arbeitsflüssigkeitspumpe eine Intervallpumpe zum intervallweisen Pumpen der Arbeitsflüssigkeit. Die Intervallpumpe pumpt die Arbeitsflüssigkeit von einem Arbeitsflüssigkeitstank durch eine flexible Leitung zur Einspritzleitung. Aus der Einspritzöffnung der Einspritzleitung tritt die Arbeitsflüssigkeit intervallartig oder impulsartig aus. Auf diese Weise lässt sich das Unterhautfett auf einfache Weise in seiner Struktur auflösen und schließlich absaugen.

Im folgenden wird unter Bezugnahme auf die Zeichnungen ein Ausführungsbeispiel der Erfindung näher erläutert.

Es zeigen:
- Fig. 1: eine Fettabsaugvorrichtung gemäß der Erfindung beim Absaugen von Unterhautfett,

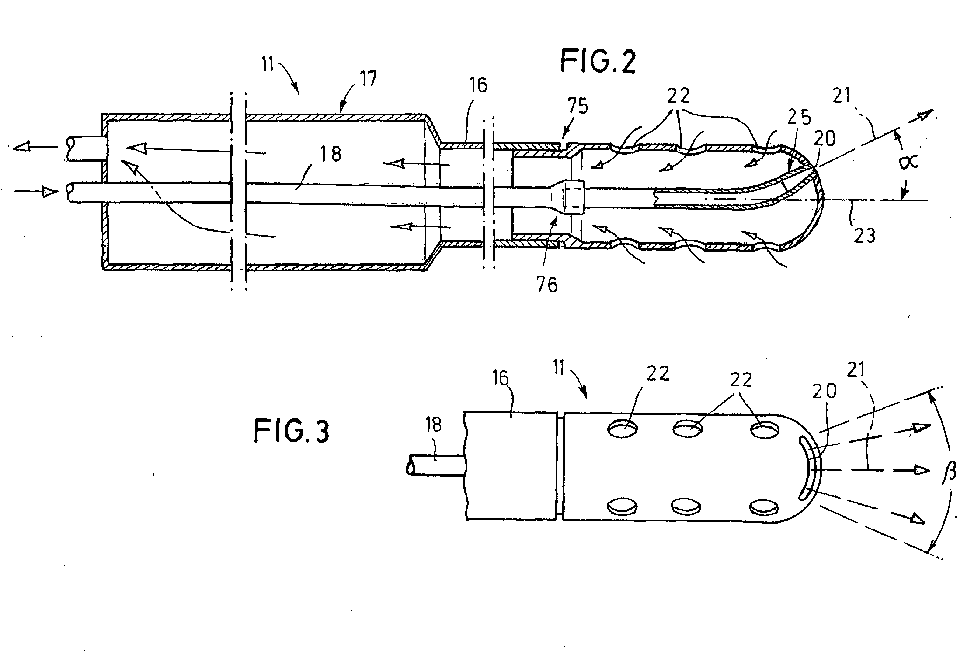

- Fig. 2: einen Längsschnitt durch die Absaugkanüle der Fettabsaugvorrichtung der Fig. 1, und
- Fig. 3: die Absaugkanüle der Fettabsaugvorrichtung der Fig. 1.

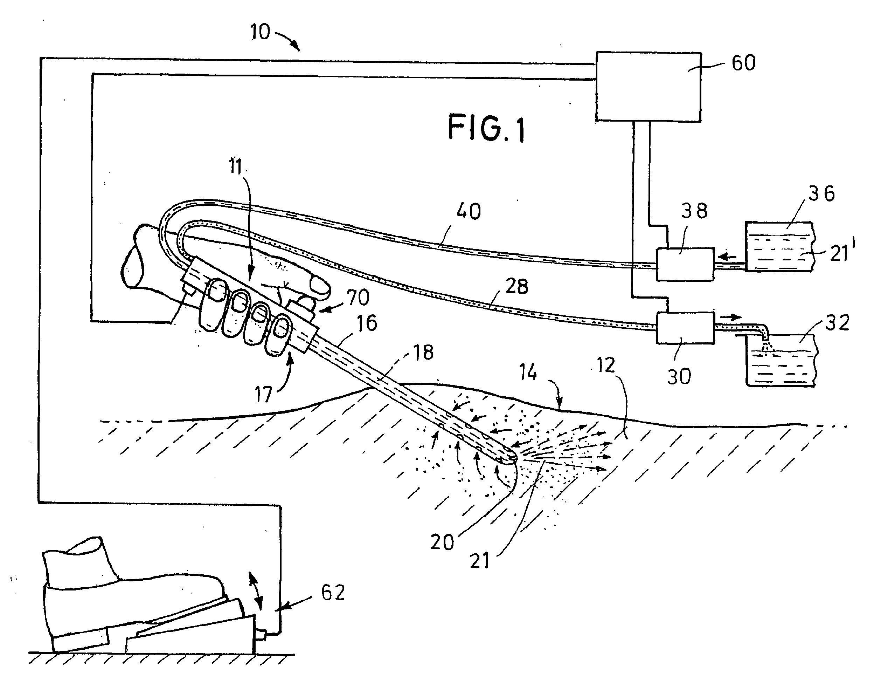

In Fig. 1 ist eine Fettabsaugvorrichtung 10 zum Entfernen von Unterhautfett 12 unterhalb einer Hautoberfläche 14 dargestellt. Die Fettabsaugvorrichtung 10 wird im Wesentlichen gebildet von einer Absauglanze 11, einem Arbeitsflüssigkeitstank 36, einem Abfalltank 32, einer Regelungsvorrichtung 60, zwei Pumpen 38,30 mit zugeordneten Flüssigkeitsleitungen 28, 40, einem Druckwähler 62 und einem Pumpenschalter 70. Die Absauglanze 11 ist durch eine kleine Schnittöffnung in der Haut 14 hindurchgeführt und taucht mit ihrem vorderen Ende in das Unterhautfett 12 unterhalb der Oberfläche der Haut 14 ein.

Die Fettabsauglanze 11 weist eine im Querschnitt kreisrunde Absaugkanüle 16 auf, innerhalb der axial und zentral in der Mitte eine Einspritzleitung 18 angeordnet ist. Die Einspritzleitung 18 weist an ihrem vorderen Ende eine trichterförmige Beschleunigungsdüse 25 und eine nach vorne geöffnete schlitzförmige Einspritzöffnung 20 auf, durch die ein Arbeitsflüssigkeits-Fächer 21 in den Unterhautfettbereich 12 eingespritzt wird. Die Einspritzöffnung 20 hat eine Schlitzbreite von ungefähr 25 µm, kann jedoch auch eine Schlitzbreite von bis zu 100 µm und mehr aufweisen. Die Länge des Einspritzöffnungsschlitzes beträgt ca. 1,2 mm kann jedoch bis zu mehreren Millimetern betragen. Der Schlitz kann gerade oder leicht gebogen ausgebildet sein, so dass ein ebener bzw. ein trichterartig gewölbter Arbeitsflüssigkeitsfächer 21 austritt. In jedem Fall ist das Verhältnis der Schlitzlänge zur Schlitzbreite größer als 10. Der Fächerwinkel β, d. h. der Fächeröffnungswinkel beträgt ungefähr 50°.

Die Absaugkanüle 16 weist am vorderen freien Ende an ihrem Umfang mehrere Absaugöffnungen 22 auf, durch die mit einem Unterdruck von 0,5 bis 0,9 bar kontinuierlich das Gemisch aus Unterhautfett und Arbeitsflüssigkeit aus dem Bereich unterhalb der Hautoberfläche 14 abgesaugt wird. Die Absauglanze 11 weist in ihrem hinteren Bereich einen Handgriff 17 auf, der gegenüber dem vorderen Bereich der Absauglanze 11 erweitert ist. Die Absauglanze 11 hat in ihrem vorderen Bereich einen Außendurchmesser von ungefähr 4,0 mm. Das vordere freie Ende der Absaugkanüle 16 besteht aus Edelstahl oder aus Saphir.

Die Arbeitsflüssigkeit 21' wird aus dem Arbeitsflüssigkeitstank 36 mit einer Arbeitsflüssigkeitspumpe 38 über eine flexible Leitung 40 in die Einspritzleitung 18 der Absauglanze 11 gepumpt. Die Pumpe 38 läuft kontinuierlich, kann jedoch auch im Intervallbetrieb betrieben werden, so dass die Arbeitsflüssigkeit in Intervallen oder impulsartig eingespritzt wird. Die Arbeitsflüssigkeit wird durch die schlitzförmige Einspritzöffnung 20 mit einem Überdruck von ca. 10-100 bar eingespritzt. Dadurch werden Fettgewebe und Fett mechanisch aus dem Unterhautfett gelöst und zerkleinert, jedoch werden dabei keine Gefäße zerstört. Der Verbrauch an Arbeitsflüssigkeit bei Schlitzmaßen von 0,025 mm x 1,2 mm beträgt ungefähr 5 l/ h. Die Arbeitsflüssigkeit kann schmerzstillende Substanzen enthalten, jedoch auch aus reiner Kochsalzlösung bestehen.

Die Absaugkanüle 16 ist mit einer flexiblen Leitung 28 mit einer Unterdruck erzeugenden Saugpumpe 30 verbunden, durch die die abgesaugte Flüssigkeit kontinuierlich in einen Abfalltank 32 gepumpt wird.

An dem Handgriff 17 ist ein Pumpenschalter 70 angeordnet, der mit einer Steuerleitung mit der Regelungsvorrichtung 60 verbunden ist. Durch Betätigen des Pumpenschalters 70 werden die Arbeitsflüssigkeitspumpe 38 und die Saugpumpe 30 eingeschaltet, bei Loslassen des Pumpenschalters 70 werden beide Pumpen 30,38 wieder abgeschaltet. Auf diese Weise ist eine einfache Kontrolle des Betriebes der Fettabsaugvorrichtung 10 möglich und ein schnelles Abschalten zur Vermeidung von unerwünschten Abtragungen jederzeit möglich.

Der Ausgangsdruck der Arbeitsflüssigkeitspumpe 38 wird durch den als Fußpedal ausgebildeten. Druckwähler 62 eingestellt, der ebenfalls mit einer Steuerleitung mit der Regelungsvorrichtung 60 verbunden ist. Mit dem Fußpedal-Druckwähler 62, lässt sich der Ausgangsdruck und/oder die Flussrate der Arbeitsflüssigkeitspumpe 38 nach Belieben des Behandlers jederzeit verstellen und anpassen. Durch die Regelungsvorrichtung 60 wird die Saugleistung der Saugpumpe 30 stets in fester Abhängigkeit von der mit dem Druckwähler 62 gewählten Pumpleistung der Arbeitsflüssigkeitspumpe 38 geregelt. Dies bedeutet, dass bei hoher Pumpleistung der Arbeitsflüssigkeitspumpe 38 auch die Saugleistung der Saugpumpe 30 entsprechend hoch eingestellt ist. Hierdurch wird sichergestellt, dass jederzeit ungefähr so viel Arbeitsflüssigkeits-Unterhautfett-Gemisch abgesaugt wird, wie gleichzeitig an Arbeitsflüssigkeit eingespritzt und an Unterhautfett gelöst wird. Dadurch wir sichergestellt, dass die entsprechende Hautpartie nicht durch Arbeitsflüssigkeit aufgepumpt wird und dass das Ergebnis des Fettabsaugens sofort erkennbar ist. Der Unterdruck der Saugpumpe 30 kann auf einen Maximalunterdruck begrenzt sein, so dass ein Festsaugen der Absaugkanüle im Unterhautgewebe ausgeschlossen ist.

Der Winkel α der Einspritzöffnung 20 zur Längsachse der Einspritzleitung 18 bzw. der Absauglanze 11 beträgt ungefähr 18°. Der Fächerwinkel β, d.h. der Öffnungswinkel der Einspritzöffnung beträgt ungefähr 50°. Sowohl die Absaugkanüle 16 als auch die Einspritzleitung 18 weisen jeweils ein Kupplungsteil 75,76 auf, so dass das vordere Ende der Absaugkanüle 16 und der Einspritzleitung 18, die zusammen eine Lanzenspitze 80 bilden, auf einfache Weise austauschbar sind. Auf diese Weise kann die Lanzenspitze 80 stets mit einfachen Mitteln ausgetauscht werden, beispielsweise um eine Einspritzöffnung mit einer anderen Schlitzform, einem anderen Öffnungswinkel, einem anderen Anstellwinkel α etc. zu montieren.

## Patentansprüche

1. Fettabsaugvorrichtung zum Entfernen von Unterhautfett (12), mit
einer Absaugkanüle (16) mit einer Absaugöffnung (22) zum Absaugen des Unterhautfettes (12), und
einer an der Absaugkanüle (16) vorgesehenen Einspritzleitung (18) mit einer Einspritzöffnung (20) zum Einspritzen einer Arbeitsflüssigkeit, wobei
die Einspritzöffnung (20) am vorderen Ende der Einspritzleitung (18) angeordnet und schlitzförmig ausgebildet ist, so dass die Arbeitsflüssigkeit in Form eines Fächers (21) austritt,
**dadurch gekennzeichnet,**
**dass** die Schlitzbreite der Einspritzöffnung (20) kleiner als 1,0 mm ist, und
**dass** die schlitzartige Einspritzöffnung (20) in einem Winkel (α) zwischen 3° und 45° geneigt zu der Längsachse (23) der Einspritzleitung (18) angeordnet ist, so dass der Arbeitsflüssigkeits-Fächer (21) unter diesem Winkel (α) zu der Einspritzleitungs-Längsachse (23) austritt.

2. Fettabsaugvorrichtung nach Anspruch 1, **dadurch gekennzeichnet, dass** die Einspritzleitung (18) an ihrem einspritzöffnungsseitigen Ende eine trichterförmige Beschleunigungsdüse (25) aufweist.

3. Fettabsaugvorrichtung nach Anspruch 1 oder 2, **dadurch gekennzeichnet, dass** eine Arbeitsflüssigkeitspumpe (38) zum Pumpen der Arbeitsflüssigkeit zu der Einspritzöffnung (20) vorgesehen ist, wobei der Ausgangsdruck der Arbeitsflüssigkeitspumpe (38) durch einen Druckwähler (62) einstellbar ist.

4. Fettabsaugvorrichtung nach einem der Ansprüche 1 - 3, **dadurch gekennzeichnet, dass** eine Saugpumpe (30) zum Absaugen des Unterhautfettes (12) und eine Regelungsvorrichtung (60) vorgesehen sind, die die Saugleistung der Saugpumpe (30) in Abhängigkeit von der Pumpleistung der Arbeitsflüssigkeitspumpe (38) regelt.

5. Fettabsaugvorrichtung nach einem der Ansprüche 1 - 4, **dadurch gekennzeichnet, dass** ein Handgriff (17) zum Halten der Absaugkanüle und der Einspritzleitung (18) vorgesehen ist, wobei an dem Handgriff (17) ein Pumpenschalter (70) zum Schalten der Arbeitsflüssigkeitspumpe (38) vorgesehen ist.

6. Fettabsaugvorrichtung nach einem der Ansprüche 1 - 5, **dadurch gekennzeichnet, dass** an der Absaugkanüle ein Ultraschallgenerator zur Gewebelockerung angeordnet ist.

7. Fettabsaugvorrichtung nach einem der Ansprüche 1 - 6, **dadurch gekennzeichnet, dass** an der Absaugkanüle ein Laser zur Gewebeerwärmung vorgesehen ist.

8. Fettabsaugvorrichtung nach einem der Ansprüche 1 - 7, **dadurch gekennzeichnet, dass** die Arbeitsflüssigkeitspumpe (38) eine Intervallpumpe zum intervallweisen Pumpen der Arbeitsflüssigkeit (21') ist.

9. Fettabsaugvorrichtung nach einem der Ansprüche 1 - 8, **dadurch gekennzeichnet, dass** die Einspritzleitung (18) mit der Einspritzöffnung (20) axial beweglich zu dem Handgriff (17) ist und ein Axialantrieb vorgesehen ist, der die Einspritzleitung (18) mit der Einspritzöffnung (20) axial oszillierend antreibt.

10. Fettabsaugvorrichtung nach einem der Ansprüche 1 - 9, **dadurch gekennzeichnet, dass** die Einspritzleitung (18) zentral innerhalb der Absaugkanüle (16) angeordnet ist und die Absaugkanüle (16) mehrere über den Umfang verteilte Absaugöffnungen (22) aufweist.

11. Fettabsaugvorrichtung nach einem der Ansprüche 1 - 10, **dadurch gekennzeichnet, dass** die schlitzartige Einspritzöffnung (20) in einem Winkel (α) zwischen 10° und 30°, insbesondere in einem Winkel (α) von ungefähr 18° geneigt zu der Längsachse (23) der Einspritzleitung (18) angeordnet ist.

12. Fettabsaugvorrichtung nach einem der Ansprüche 1 - 11, **dadurch gekennzeichnet, dass** die Schlitzbreite der Einspritzöffnung (20) kleiner als 0,1 mm ist.

## Claims

1. A liposuction device for removing subcutaneous fat (12), comprising
a suction cannula (16) with a suction opening (22) for sucking the subcutaneous fat (12), and
an injection line (18) provided at the suction cannula (16), with an injection opening (20) for injecting a working fluid,
the injection opening (20) being arranged at the front end of the injection line (18) and has a slit-shaped configuration so that the working fluid exits in the shape of a fan (21),
**characterized in**
**that** the slit width of the injection opening (20) is smaller than 1.0 mm, and
**that** the slit-shaped injection opening (20) is arranged so as to be inclined to the longitudinal axis (23) of the injection line (18) at an angle (α) between 3° and 45° so that the working fluid fan (21) exits at this angle (α) with respect to the longitudinal axis (23) of the injection line.

2. The liposuction device according to claim 1, **characterized in that** the injection line (18) comprises a funnel-shaped acceleration nozzle (25) at its injection opening-side end.

3. The liposuction device according to one of claims 1 or 2, **characterized in that** a working fluid pump (38) for pumping the working fluid to the injection opening (20) is provided, the delivery pressure of the working fluid pump (38) being adjustable by a pressure selector (62).

4. The liposuction device according to one of claims 1 - 3, **characterized in that** a suction pump (30) for sucking the subcutaneous fat (12) and a control device (60) controlling the suction power of the suction pump (30) in dependence on the pump power of the working fluid pump (38) are provided.

5. The liposuction device according to one of claims 1 - 4, **characterized in that** a handle (17) for holding the suction cannula and the injection line (18) is provided, a pump switch (70) for switching the working fluid pump (38) being provided at the handle (17).

6. The liposuction device according to one of claims 1 - 5, **characterized in that** an ultrasonic generator for loosening the tissue is arranged at the suction cannula.

7. The liposuction device according to one of claims 1 - 6, **characterized in that** a laser for heating the tissue is provided at the suction cannula.

8. The liposuction device according to one of claims 1 - 7, **characterized in that** the working fluid pump (38) is an interval pump for intermittently pumping the working fluid (21').

9. The liposuction device according to one of claims 1-8, **characterized in that** the injection line (18) with the injection opening (20) is axially movable with respect to the handle (17), and an axial drive is provided that drives the injection line (18) with the injection opening (20) in an axially oscillating manner.

10. The liposuction device according to one of claims 1 - 9, **characterized in that** the injection line (18) is arranged centrally within the suction cannula (16) and the suction cannula (16) comprises several suction openings (22) distributed over the circumference.

11. The liposuction device according to one of claims 1 - 10, **characterized in that** the slit-like injection opening (20) is arranged so as to be inclined to the longitudinal axis (23) of the injection line (18) at an angle (α) between 10° and 30°, particularly at an angle (α) of about 18°.

12. The liposuction device according to one of claims 1 - 11, **characterized in that** the slit width of the injection opening (20) is smaller than 0.1 mm.

## Revendications

1. Dispositif de liposuccion pour retirer la graisse sous-cutanée (12), avec
une canule de succion (16) avec une ouverture de succion (22) pour aspirer la graisse sous-cutanée (12), et
un conduit d'injection (18) prévu sur la canule de succion (16) avec un orifice d'injection (20) pour injecter un liquide de travail, dans lequel
l'orifice d'injection (20) est disposé à l'extrémité avant du conduit d'injection (18) et est configuré en forme de fente, de sorte que le liquide de travail sont sous forme d'éventail (21),
**caractérisé en ce que**,
la largeur de fente de l'orifice d'injection (20) est inférieure à 1 mm, et
l'orifice d'injection (20) en forme de fente est disposé en étant incliné d'un angle (α) compris entre 3° et 45° par rapport à l'axe longitudinal (23) du conduit d'injection (18), de sorte que l'éventail (21) de liquide de travail sort selon cet angle (α) par rapport à l'axe longitudinal (23) du conduit d'injection.

2. Dispositif de liposuccion selon la revendication 1, **caractérisé en ce que** le conduit d'injection (18) présente à son extrémité côté orifice d'injection, une base d'accélération (25) en forme d'entonnoir.

3. Dispositif de liposuccion selon la revendication 1 ou 2, **caractérisé en ce qu'**une pompe de liquide de travail (38) est prévue pour pomper le liquide de travail vers l'orifice d'injection (20), la pression de sortie de la pompe de liquide de travail (38) étant réglable par un sélecteur de pression (62).

4. Dispositif de liposuccion selon l'une des revendications 1 à 3, **caractérisé en ce qu'**une pompe d'aspiration (30) est prévue pour aspirer la graisse sous-cutanée (12), ainsi qu'un dispositif de régulation (60) qui régule la puissance d'aspiration de la pompe d'aspiration (30) en fonction du débit de la pompe de liquide de travail (38).

5. Dispositif de liposuccion selon l'une des revendications 1 à 4, **caractérisé en ce qu'**une poignée (17) est prévue pour maintenir la canule de succion et le conduit d'injection (18), un commutateur de pompe (70) étant prévu sur la poignée (17) pour la commutation de la pompe de liquide de travail (38).

6. Dispositif de liposuccion selon l'une des revendications 1 à 5, **caractérisé en ce qu'**un générateur à ultrasons est disposé sur la canule de succion pour le relâchement des tissus.

7. Dispositif de liposuccion selon l'une des revendications 1 à 6, **caractérisé en ce qu'**un laser est prévu sur la canule de succion pour le réchauffement des tissus.

8. Dispositif de liposuccion selon l'une des revendications 1 à 7, **caractérisé en ce que** la pompe de liquide de travail (38) est une pompe intermittente pour le pompage intermittent du liquide de travail (21').

9. Dispositif de liposuccion selon une des revendications 1 à 8, **caractérisé en ce que** le conduit d'injection (18) avec l'orifice d'injection (20) est axialement mobile par rapport à la poignée (17), et il est prévu une commande axiale qui actionne de façon axialement oscillante le conduit d'injection (18) avec l'orifice d'injection (20).

10. Dispositif de liposuccion selon l'une des revendications 1 à 9, **caractérisé en ce que** le conduit d'injection (18) en position centrale à l'intérieur de la canule de succion (16), et la canule de succion (16) comprend plusieurs ouvertures d'aspiration (22) réparties sur la périphérie.

11. Dispositif de liposuccion selon l'une des revendications 1 à 10, **caractérisé en ce que** l'orifice d'injection (20) en forme de fente est disposé en étant incliné d'un angle (α) compris entre 10° et 30°, en particulier d'un angle (α) d'environ 18°, par rapport à l'axe longitudinal (23) du conduit d'injection (18).

12. Dispositif de liposuccion selon l'une des revendications 1 à 11, **caractérisé en ce que** la largeur de fente de l'orifice d'injection (20) est inférieure à 0,1 mm.
